# EUROPEAN PATENT APPLICATION

(11) **EP 0 834 296 A1**
(43) Date of publication of application: **08.04.1998**
(21) Application number: 96115751.8
(22) Date of filing: 02.10.1996
(51) Int. Cl.: A61F 13/15

(54) **Disposable absorbent article having a profiled absorbent structure being capable of self-shaping in use**

(71) Applicant: THE PROCTER & GAMBLE COMPANY, Cincinnati, Ohio 45202 (US)
(72) Inventor: Carlucci, Giovanni, 66100 Chieti (IT); D'Alessio, Nicola, 65126 Pescara (IT); Giorgini, Gennaro, 64026 Roseto (IT); Tamburro, Maurizio, 65100 Pescara (IT)
(74) Representative: Canonici, Jean-Jacques

(57) **Abstract**

A disposable absorbent article for wearing adjacent a body discharge area having a longitudinal centreline and a lateral centreline orthogonal thereto and defining longitudinal and lateral directions respectively. The disposable absorbent article comprises a liquid pervious topsheet, a backsheet joined to said topsheet and an absorbent core intermediate the backsheet and the topsheet. The absorbent core comprises an expanding layer for expanding the article into a tridimensional structure while being worn by a user. The expanding layer is activated by body fluids and has a predetermined profile in Z-direction in the dry state prior to use. The expanding layer is capable, upon activation by body fluids, of expanding into the predetermined wet profile.

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to disposable absorbent sanitary napkins, catamenials, incontinence inserts, pantiliners and diapers comprising an expanding layer for expanding the article into a tridimensional structure while being worn by a user. The expanding layer is activated by body fluids and provides the article with a self-shaping capability during the use. The expanding layer further has a predetermined profile in z-direction prior to use, and is capable of expanding into the predetermined wet profile upon activation by body fluids.

### BACKGROUND OF THE INVENTION

In their basic form, disposable absorbent articles comprise an absorbent core interposed between a pervious body-contacting element (alternatively referred to as a topsheet or an overwrap) and an impervious protective barrier (alternatively referred to as a backsheet). The absorbent element is, of course, intended to receive and contain the fluids discharged from the body. The body-contacting element is intended to provide comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles as sanitary napkins are provided with an adhesive attachment means for securing the device to the inner crotch area of the user's undergarments.

An improvement in the comfort and convenience of such absorbent articles has been in the development of absorbent articles that achieve a better fluid interception by means of a closer contact of the absorbent article itself with the body of the wearer.

While previously known absorbent articles do perform their intended function, each conventional design can be further improved in one or more of absorbency of body fluids, protection of the user's garments from soiling, physical comfort to the user, and/or capability of providing an anatomically shaped configuration for a closer body contact.

With respect to disposable sanitary napkins several attempts have been made in the art to improve body contact with the wearer, and hence absorb fluids upon discharge and thereby minimize soiling by providing a sanitary napkin having an anatomically shaped configuration, particularly including those that are raised upwardly or humped in their medial portions so as to be near or in contact with the pudendal region when worn.

On female users this type of sanitary napkins attempts to contact and absorb menses immediately as it leaves the vestibule.

Some articles have been also described in which an anatomically shaped configuration is provided during the wearing time, with the advantage of a better fit to the anatomy.

U.S. Patent No. 3,736,931 discloses a sanitary napkin having an outer non-compressed layer of fluid absorbent material and an inner core of highly compressed fluid absorbent material which is at least partially enclosed therein. The napkin preferably is V-shaped in cross section and is arch-shaped in its longitudinal direction by die compression. When the napkin is worn the fluid directs first into the inner compressed layer so as to cause it to swell and to expand the outer non-compressed layer in all directions, thereby adjusting itself to each wearer.

The sanitary napkin expands upon fluid absorption and may adjust itself to the user's anatomy, but since it is not flat prior to use it may be cumbersome to package and to handle; moreover, the expansion takes place mainly in lateral direction, so achieving an effective seal against the inner side of the thighs and at both sides of the vaginal orifice; therefore the structure is not capable of achieving a close contact with the user's anatomy, particularly in that it does not bring the absorbent element in direct contact with the point of release of the fluid.

According to U.S. Patent No. 3,512,530 a sanitary napkin is described in which a compressed regenerated cellulose sponge layer is combined to a larger fibrous cellulose layer to form a multiple ply absorbent core. The compressed regenerated cellulose sponge layer is positioned over the fibrous layer, and it is typically centered about it; it is intended as the primary absorbent element of the sanitary napkin, while the fibrous layer acts as a secondary or back up absorber.

The sanitary napkin may be therefore very thin prior to use, as compared to other sanitary products having the same absorbent capacity.

Although the compressed regenerated cellulose sponge layer is capable of expanding in Z-direction upon fluid absorption, the structure described is not particularly suitable to provide an effective body contact with the wearer's anatomy and might cause discomfort to the user due to the characteristics of the compressed regenerated cellulose sponge material, particularly when it is dry.

EP Patent 293 208 B1 describes the use of multiple layers of compressed regenerated cellulose sponge sheets in a sanitary napkin as the sole absorbent material instead of the usual cellulose pulp absorbent core in order to obtain an absorbent article of improved strength and shape retainability in wet conditions, as compared to traditional absorbent articles with fluff cores that tend to be broken or to form lumps in use.

The sheets are provided with slits in order to enhance their flexibility, with a better comfort for the user, and to increase the fluid absorbing area.

The sanitary napkin described in EP patent 293 208 B1 has a structure that is not specifically intended to provide a self shaping capability during the use taking advantage of the swelling of the absorbent material, but rather a better strength when wetted than articles using conventional, fluff-based absorbent cores, and a better flexibility and absorbency rate as compared to articles using the same compressed regenerated cellulose sponge material.

In European application EP 96106721.2, filed on 29 April 1996, absorbent articles are described which comprise means for expanding the article into a tridimensional structure while being worn by the user. The means is activated by body fluids and comprises a sheet of compressed regenerated cellulose sponge.

The absorbent articles described in the above mentioned application are capable of providing an anatomically shaped configuration for a close body contact which is achieved during the use upon activation by absorbed body fluids, and are comfortable for the wearer and easy to produce and to package. Their performances can be improved in terms of a better capability of conforming to the user's anatomy.

It is therefore an object of the present invention to provide an absorbent article capable of providing a closer body contact which is achieved during the use upon activation by absorbed body fluids, while it is comfortable for the wearer, easy to produce and to package, and capable of achieving easily and very rapidly upon activation by body fluids even complex anatomically shaped configurations.

It is a further object of the present invention to provide an absorbent article which has a better capability of achieving a closer body contact during the use, even in the period of time before activation by body fluids, while preferably still being substantially flat and thin prior to use.

### SUMMARY OF THE INVENTION

The present invention relates to disposable absorbent articles for wearing adjacent a body discharge area, which are preferably substantially flat prior to use. The disposable absorbent article has a longitudinal centreline and a lateral centreline orthogonal thereto that define longitudinal and lateral directions respectively, and a Z-direction that is orthogonal to both of them. The disposable absorbent article comprises a liquid pervious topsheet, a backsheet, preferably liquid impervious, joined to said topsheet, and an absorbent core intermediate the topsheet and the backsheet. The term "substantially flat", as used herein, refers to articles that have their main extension in the X-Y plane. The absorbent core comprises an expanding layer for expanding the article into a tridimensional structure while being worn by a user, wherein the expanding layer is activated by body fluids. The expanding layer has a predetermined profile in the Z-direction in the dry state prior to use, and is capable of expanding, upon activation by body fluids, into the predetermined wet profile.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a sanitary napkin according to the present invention;
FIG. 2 is a cross-sectional view of the sanitary napkin shown in FIG. 1 as taken along a section line corresponding to the transverse centreline A-A;
FIG. 3 is a cross-sectional view similar to that of FIG. 2, showing the sanitary napkin expanded into a tridimensional structure after activation during wear;
FIGS. 4a and 4b are a cross-sectional view taken along the transverse centreline A-A of the expanding layer showing alternate embodiments of the predetermined profile;
FIG. 5 is a cross-sectional view of another embodiment of a sanitary napkin according to the present invention;
FIG. 6 is a cross-sectional view similar to that of FIG. 2, showing the sanitary napkin expanded into a tridimensional structure after activation during wear.

### DETAILED DESCRIPTION OF THE INVENTION

This invention relates to a disposable absorbent article which exhibits absorbency for bodily fluids, the protection of the user's garments from soiling, improved physical comfort to the user, and which is easy to produce and to package. The disposable absorbent article is described below by reference to a sanitary napkin or catamenial. The term "sanitary napkin", as used herein, refers to an article which is worn by females adjacent to the pudendal region and which is intended to absorb and contain the various body fluids which are discharged from the body (e.g., vaginal discharges, menses, and/or urine) and which is intended to be discarded after a single use. The disposable absorbent article is preferably substantially flat prior to use.

The term "substantially flat", as used herein, refers to articles that have their main extension in one plane. In a preferred embodiment a substantially flat article will have an absorbent core that extends substantially in the X-Y directions, with the dimension which extends in the Z-direction being substantially smaller than the two other dimensions extending in the X and in the Y directions, respectively. This comprises an absorbent core having a thickness that is not constant or, in other words, being shaped in a direction which is orthogonal to the absorbent core itself, provided that the above condition is satisfied. This does not exclude, also, a general curvature of the absorbent core. It will be apparent to the man skilled in the art to which extent products can deviate from absolute flat shape and still benefit from the during the use shaping according to the present invention.

Sanitary napkins with longitudinal side cuffs, which may be optionally elasticated, and sanitary napkins with a moderate curvature are therefore within the scope of the present invention, and their absorbent core can be preferably substantially flat, according to the definition given above.

The term "predetermined profile", as used herein, refers to an expanding layer that comprises zones that, upon activation by body fluids, are capable of expanding more than other zones according to an actual, desired profile, which substantially corresponds to the predetermined profile of the expanding layer in its dry state. The profile achieved by the expanding layer after expansion upon activation by body fluid is the "predetermined wet profile" referred to herein. The term "predetermined profile", therefore, is associated in this context with an actual particular contour of the expanding layer when seen in cross-section in its dry state before activation by body fluids.

The term "use", as used herein, refers to the period of time that starts when the absorbent article is actually put in contact with the anatomy of the user.

The terms "joined" or "affixed", as used herein, encompasses configurations whereby a first member is directly connected to a second member and configurations whereby a first member is indirectly connected to a second member by connecting the first member to intermediate members which in turn are connected to the second member.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

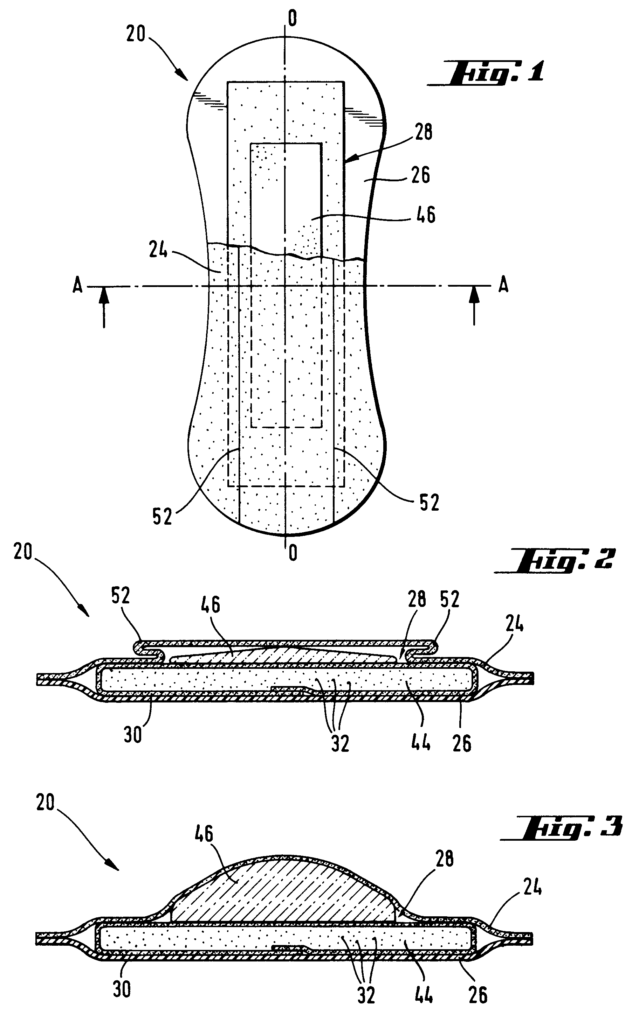

In FIGS. 1 and 2, one preferred embodiment of a sanitary napkin 20 of the present invention is shown. Fig. 1 is a plan view of the sanitary napkin 20 of the present invention in its flat-out state prior to use with portions of the structure being cut-away to more clearly show the construction of the sanitary napkin 20 and with the portion of the sanitary napkin 20 which faces or contacts the wearer oriented towards the viewer. As shown in FIGS. 1 and 2, the sanitary napkin 20 preferably comprises a liquid pervious topsheet 24, a liquid impervious backsheet 26 joined to the topsheet 24, and an absorbent core 28 intermediate the topsheet 24 and the backsheet 26; the absorbent core 28 comprises an expanding layer 46 for expanding the sanitary napkin into a tridimensional structure while being worn by a user.

In a preferred embodiment of the present invention illustrated in FIGS. 1 to 3, the absorbent core 28 comprises the expanding layer 46 and a separate, substantially non expanding absorbent element 44 joined together and in face to face relationship to each other, the expanding layer 46 being positioned between the topsheet 24 and the absorbent element 44.

The absorbent element 44 and the expanding layer 46 may be associated in any suitable manner to form the absorbent core 28. Suitable manners include, but are not limited to, associating the absorbent element 44 and the expanding layer 46 with adhesives such as by spray-gluing or by applying lines or spots of adhesive between them. Alternatively, or additionally, the association between the layers may be achieved by fibre entanglement or by a plurality of discrete fusion bonds.

Alternatively, the expanding layer 46 may constitute the entire absorbent core 28.

The absorbent capacity of the absorbent core 28 should be compatible with the intended body fluid loading for the sanitary napkin 20. Further, the overall absorbent capacity of the absorbent core 28 may be varied to accommodate wearers ranging in the expected amount of body fluid volume. For instance, a different absorbent capacity may be utilized for sanitary napkins intended for day time use as compared with those intended for night time use, or for sanitary napkins intended for use by teenage females as compared with those intended by more mature women.

The sanitary napkin 20 has two surfaces, a body contacting or facing surface, and a garment facing or contacting surface. The absorbent core 28 has corresponding body facing and garment facing surfaces. The sanitary napkin 20 has two centrelines, a longitudinal centreline O-O and a transverse centreline A-A orthogonal thereto. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral", as used herein, are interchangeable, and refer to a line, axis, or direction which lies within the plane of the sanitary napkin 20 and is generally perpendicular to the longitudinal direction. The Z-direction is orthogonal both the longitudinal and lateral directions of the sanitary napkin 20 and extends outwardly from the plane of the sanitary napkin 20, which is defined by the longitudinal centreline O-O and the lateral centreline A-A. The term "longitudinally oriented" refers to a direction ±45 degrees of the longitudinal direction in the plane of the sanitary napkin 20; the term "laterally oriented" refers similarly to any other direction in the plane of the sanitary napkin 20.

The long edges of the sanitary napkin 20, which are aligned with the longitudinal centreline O-O, are the longitudinal side margins of the sanitary napkin 20. The ends of the sanitary napkin 20 joining the longitudinal side margins are the transverse ends of the sanitary napkin 20. Collectively the longitudinal side margins and transverse ends of the sanitary napkin 20 define its periphery. Similarly, the absorbent core 28 of the sanitary napkin 20 has a periphery defined by alternatively disposed longitudinal side margins and transverse ends.

Tridimensional structures of the sanitary napkin 20 are those in which the sanitary napkin structure is caused to expand, at least partially, in the Z-direction, in order to more closely conform to the user's anatomy. Said expansion preferably takes place in a direction that goes from the garment facing surface towards the body facing surface of the sanitary napkin 20. Particularly preferred are tridimensional structures with a convex upward configuration that are inclusive of, but not limited to, inverted U-shapes or inverted V-shapes as seen in lateral direction, with "convex upward configuration" being meant a structure of the sanitary napkin that is convex on its body facing surface. With these configurations the cross-sectional contour of the central portion of the sanitary napkin more closely matches the labia of the typical wearer. Alternative preferred tridimensional structures of the sanitary napkin 20 are W-shapes or inverted W-shapes as seen in lateral direction.

The topsheet 24 is compliant, soft feeling, and non-irritating to the wearer's skin. Further, the topsheet 24 is liquid pervious, permitting liquid to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibres (e.g., wood or cotton fibers), synthetic fibres (e.g., polymeric fibres such as polyester, polypropylene, or polyethylene fibres); or from a combination of natural and synthetic fibres.

A preferred topsheet comprises an apertured formed film. Apertured formed films are preferred for the topsheet because they are pervious to body fluids and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer.

Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. A preferred topsheet for the absorbent article of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface were not hydrophilic so as to diminish the likelihood that menstrual fluid will flow off the topsheet rather than flowing into and being absorbed by the absorbent core.

The topsheet of the present invention is preferably capable of expanding as the sanitary napkin 20 expands in a tridimensional structure upon absorption of body fluids. This may be achieved when the topsheet is made of a material that is intrinsically extensible under the forces exerted by the expanding layer 46. In a preferred embodiment illustrated in FIGS. 1 and 2 the topsheet 24 is provided with two pleats or folds 52 symmetrically positioned at both sides of the longitudinal centreline O-O and substantially parallel to it. As shown in FIG. 2 the topsheet 24 in each pleat or fold 52 is folded twice on itself toward the longitudinal side margins of the sanitary napkin 20. A single pleat or fold or, alternatively, more than two folds may be also comprised in the topsheet 24 without departing from the scope of the present invention; the pleats or folds may be generally longitudinally or laterally oriented.

The backsheet 26 is impervious to liquids (e.g., menses and/or urine) and is preferably manufactured from a thin plastic film, although other flexible liquid impervious materials can also be used. In use, the backsheet 26 is interposed between the absorbent core 28 and the user's undergarments. The function of the backsheet 26 is to prevent exudates which may be expelled from or which inadvertently bypass the absorbent core 28 from contacting and soiling the user's undergarments. The backsheet 26 can thus comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm to about 0.015 mm. The backsheet 26 is preferably embossed and/or matte finished to provide a more clothlike appearance. Further, the backsheet can permit vapors to escape from the absorbent element 26 (i.e., breathable) while still preventing exudates from passing through the backsheet.

In a preferred embodiment of the present invention the sanitary napkin 20 is also provided with a panty fastening means, not shown in the figures for clarity, which provides means to attach the article to an undergarment. For example the panty fastening means may comprise a mechanical fastener such as hook and loop fasteners such as marketed under the tradename VELCRO, snaps or holders. Alternatively, the sanitary napkin 20 is fastened to the undergarment by means of panty fastening adhesive on the backsheet 26. The panty fastening adhesive provides a means for securing the sanitary napkin 20 to the panty and preferably a means for securing the sanitary napkin 20 when soiled to the fold and wrap package for convenient disposal. Typically, at least a portion of the garment facing surface of the backsheet 26 is coated with adhesive to form the panty fastening adhesive. Any adhesive or glue used in the art for such purposes can be used for the panty fastening adhesive herein. Pressure sensitive adhesives are most preferred. Suitable adhesives include Century A-305-IV manufactured by the Century Adhesives Corporation of Columbus, Ohio, and Instant LOK 34-2823 manufactured by the National Starch and Chemical Company of Bridgewater, New Jersey, 3 Sigma 3153 manufactured by 3 Sigma and Fuller H-2238ZP manufactured by the H.B. Fuller Co.

The panty fastening adhesive is typically applied to the backsheet by slot coating or spraying in various distribution patterns, such as e.g. continuous or discontinuous strips, intermittent dots, random patterns spirals.

The panty fastening adhesive is typically covered with a removable release paper or film in order to prevent the adhesive from drying out or adhering to another surface other than the panty prior to use. Any commercially available release paper or film may be used. Suitable examples include BL 30MG-A SILOX EI/O and BL 30 MG-A SILOX 4 P/O available from Akrosil Corporation.

If present, as illustrated in FIGS. 1 to 3, the substantially non expanding absorbent element 44 of the absorbent core 28 can comprise any absorbent means which is generally compressible, resilient, non-irritating to the wearer's skin and capable of absorbing and containing body fluids. The absorbent element 44 may be manufactured from a wide variety of liquid absorbent materials commonly used in disposable sanitary napkins, and other disposable absorbent articles. Examples of suitable absorbent materials include comminuted wood pulp (which is generally referred to as airfelt), creped cellulose wadding, modified cross-linked cellulose fibres (such as those described in U.S. Patent No. 5,217,445 issued to Young, et al. on June 8, 1993), capillary channel fibres (that is, fibres having intra-fibre capillary channels such as those described in U.S. Patent No. 5,200,248 issued to Thompson, et al. on April 6, 1993), absorbent foams (such as those described in U.S. Patent No. 5,260,345, issued to DesMarais, et al. on November 9, 1993 and U.S. Patent No. 5,268,244 issued to DesMarais, et al. on December 7, 1993), thermally bonded airlaid materials (such as those material described in U.S. Patent Application Serial No. 08/141,156, entitled "Catamenial Absorbent Structures Having Thermally Bonded Layers For Improved Handling of Menstrual Fluids and Their Use In Catamenial Pads Having Improved Fit and Comfort" filed in the name of Richards, et al. on October 21, 1993), absorbent sponges, synthetic staple fibres, polymeric fibres, hydrogel-forming polymer gelling agents, peat moss, or any equivalent materials or combinations of materials. Suitable absorbent cores comprising foams are described in European Applications 0 598 833, 0 598 823 and 0 598 834.

In the embodiment illustrated in FIGS. 1 to 3, the substantially non expanding absorbent element 44 of the absorbent core 28 comprises an absorbent layer 30 made of a thermally bonded airlaid material longitudinally folded twice on itself and comprising particles 32 of absorbent gelling material therebetween.

As shown in FIGS. 1 and 2 the absorbent core 28 comprises an expanding layer 46 having a predetermined profile in Z-direction in the dry state prior to use for expanding the sanitary napkin 20 into the desired tridimensional structure while the sanitary napkin 20 is being worn. In an embodiment illustrated in FIGS. 1 to 3 the expansion and the final shaping of the sanitary napkin 20 into the tridimensional structure is provided by the swelling, substantially in Z-direction, and into a predetermined wet profile, of the material that constitutes the expanding layer 46 and that is activated during wear by the absorption of body fluids.

The expanding layer 46 can comprise any material that is capable of such swelling in order to shape the sanitary napkin 20 into the desired tridimensional structure.

After the absorption of body fluids and the subsequent swelling into the predetermined wet profile, the material of the expanding layer 46 must be soft, compliant, conformable and resilient. It must be compressible such that it will deform under the relatively small forces that are experienced during normal use. In addition to be compressible, the material of the expanding layer 46 must be flexible and conformable after swelling so it can provide improved fit through the topsheet 24 into and around the wearer's labia and perineum when the tridimensional structure is formed during the wearing time. The ability to follow the topography of the anatomy will provide intimate contact with the exposed genitalia of the female user. This helps provide better fluid transfer from the user into the expanding layer 46. While these characteristics of the expanding layer 46 allow for improved fit, they also cause the product to be both soft and comfortable for the wearer.

It is preferred that the expanding layer 46 forms at least part of the body facing surface of the absorbent core 28. In the embodiment illustrated in FIGS. 1 and 2 the expanding layer 46 is positioned over the absorbent element 44, in face to face relationship with it; it is rectangular and preferably narrower and shorter than the absorbent element 44, as illustrated in FIG. 1, being centered about both the longitudinal and transverse centrelines O-O and A-A. As an alternative, different shapes are also possible for the expanding layer 46, e.g. an hourglass shape.

The expanding layer 46 of the absorbent core 28 has a body facing surface and a garment facing surface, and has a predetermined profile in Z-direction in the dry state prior to use.

The predetermined profile of the expanding layer 46 can be formed either by the body facing surface or by the garment facing surface of the expanding layer 46, or by both of them; in other words either of the facing surfaces of the expanding layer 46 can be profiled, i.e. not planar or not parallel to each other. Preferably the predetermined profile is formed only by the body facing surface of the expanding layer 46.

The predetermined profile in the expanding layer 46 is preferably provided by means of varying thicknesses of the expanding layer 46; the thickness of the expanding layer 46 can vary either in lateral direction, as illustrated in FIGS. 2, 4a, 4b, and 5, or in longitudinal direction, or in both. When the expansion of the expanding layer 46 into the predetermined wet profile takes place during the use of the sanitary napkin 20, substantially in Z-direction and upon activation by absorbed body fluids, it substantially occurs according to the predetermined profile of the expanding layer 46, that is to say, thicker zones of the expanding layer 46 will tend to swell in Z-direction more than thinner zones, after full uniform activation of the expanding layer by body fluids, i.e., after the expanding layer 46 has been completely activated by absorbed body fluids.

The thickness of the expanding layer 46 is therefore not constant throughout the whole surface of the expanding layer 46 itself prior to use, but varies to create said predetermined profile. The predetermined profile must be such that the expanding layer 46, upon activation by absorption of body fluids during the wearing time, expands substantially in Z-direction into said predetermined wet profile in order to shape the sanitary napkin 20 into a tridimensional structure that more closely conforms to the anatomy of the user.

By carefully choosing the predetermined profile of the expanding layer 46 it is possible to have an expanding structure for the sanitary napkin 20 that is not only capable of achieving a closer conformability to the user's anatomy, but that can also provide a quicker reaction to the activation by the first, and possibly small, amount of body fluid that is absorbed. This can be explained since, in addition to the self-conforming capabilities of the material itself that constitutes the expanding layer 46, the swelling of the expanding layer 46 can occur according to a profile that already exist in the structure of the expanding layer 46.

Moreover, the expanding layer 46 with the predetermined profile can show a better ability to form, upon activation by body fluids, more complex body conforming tridimensional structures, for example those having both a convex and a concave upward configuration, such as W-shapes or inverted W-shapes, as seen in lateral direction.

Preferably, the expanding layer 46 also provides the sanitary napkin 20 of the present invention with a better capability of conforming to the user's anatomy during the use and before activation by body fluids, owing to the predetermined profile that exists in the expanding layer 46 even before any swelling has taken place, and that can provide a closer body contact, while the sanitary napkin 20 is preferably substantially flat prior to use and thin at least until the activation by body fluids.

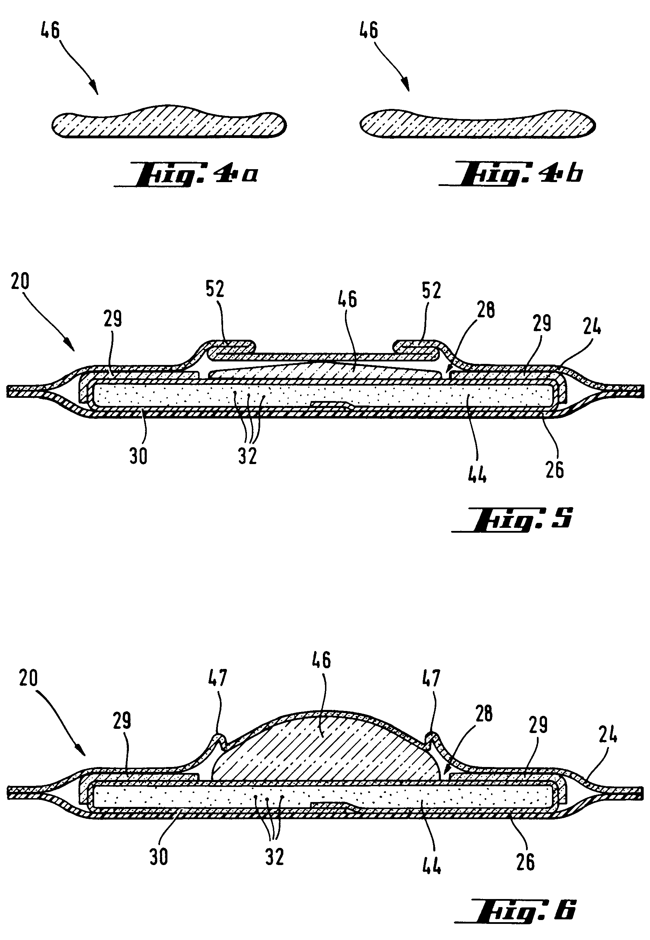

Preferred predetermined profiles of the expanding layer 46 are those that cause the expanding layer 46, upon activation by body fluids, to expand into the predetermined wet profile itself in order to more closely conform to the user's anatomy. Suitable predetermined profiles of the expanding layer 46 are those illustrated in FIGS. 2 and 5 in which the expanding layer 46 has a predetermined profile, as seen in lateral direction, such that the expanding layer is thicker along the longitudinal axis O-O, and is thinner at its longitudinal side margins; this type of predetermined profile provides the article during the use, upon activation by body fluids, and, to a lesser extent, even before said activation, with a substantially convex upward configuration. Other alternative predetermined profiles can be those illustrated in FIGS. 4a and 4b, which are capable of providing, upon activation by body fluids, W-shaped and inverted W-shaped profiles respectively, as seen in lateral direction.

Particularly, the expanding layer 46 shown in FIG. 4b can expand, upon activation by body fluids, into a predetermined wet profile that provides a sanitary article with a type of longitudinal barriers that can be beneficial for the prevention of side leakage.

Predetermined profiles for an expanding layer 46, as seen in lateral direction, need not be constant along the longitudinal centreline O-O; they can therefore vary along said longitudinal centreline O-O in order to better match the user's anatomy that typically varies from the front portion of the pudendal region, back to the perineum and to the buttocks.

The expanding layer 46 can be provided with the predetermined profile by means of any known method. The expanding layer 46 can be therefore constituted by a single layer featuring the desired predetermined profile, achieved by e.g. carving a thicker layer, or by moulding one according to the preferred profile. Alternatively, the expanding layer 46 can comprise multiple thinner layers having different shapes and dimensions, superimposed and joined to each other in order to form an expanding layer 46 with the desired profile.

The expanding layer 46 has preferably a uniform density, even though different densities can be provided in different zones of the expanding layer 46.

The behaviour of a sanitary napkin 20 comprising an expanding layer 46 with a predetermined profile is shown in FIGS. 2 and 3 in which the sanitary napkin 20 is illustrated before and after expansion of the expanding layer 46, respectively. FIG. 2 shows the sanitary napkin 20 before the first fluid absorption, with the expanding layer 46 having its predetermined profile prior to swelling. FIG. 3 shows the sanitary napkin 20 expanded into a tridimensional structure after activation of the expanding layer 46 upon absorption of a first amount of body fluid received approximately into the centre of the expanding layer 46. The first release of fluid is rapidly acquired within the expanding layer 46 and causes it to swell in Z-direction increasing its thickness, as can be seen in FIG. 3, into the predetermined wet profile. In the preferred embodiment illustrated in FIG. 3 the topsheet 24 follows the swelling of the expanding layer 46 by straightening out the pleats or folds 52, therefore without restraining the swelling.

The swelling of the expanding layer 46 upon activation by body fluids and according to the predetermined profile gives the sanitary napkin 20 an increased capacity of conforming rapidly and effectively to the user's anatomy.

As an alternate embodiment the expanding layer 46 with the predetermined profile can further comprise, on at least its body facing surface or its garment facing surface, incisions as described in European application EP 96106724.6, or apertures as described in European application EP 96106723.8, or any combination thereof, as e.g. described in European application EP 96110575.6, all applications having the same priority date of 29 April 1996.

According to further alternate embodiments the expanding layer 46 with the predetermined profile can comprise a number of smaller elements that are decoupled from one another and are also coplanar, as described in European patent application EP 96110576.4, or a number of smaller expanding elements decoupled from one another and each being capable of expanding substantially in only one direction, as described in European patent application EP 96110572.3, both applications filed on 1 July 1996.

In a preferred embodiment of the present invention the expanding layer 46 comprises a sheet of compressed regenerated cellulose sponge.

The regenerated cellulose sponge that preferably constitutes the expanding layer 46 is a material that is known in the art; examples of suitable materials are described in U.S. Patent No. 3,954,493, in French Patent Application FR-A-2,203,827, and in European Patent EP-B-0 293 208. The regenerated cellulose sponge is a sponge of a material containing a cellulose skeleton. Examples of such sponges include, in addition to sponges consisting of cellulose itself, sponges consisting of a cellulose derivative as viscose, a cellulose ether and a cellulose ester, and sponges consisting of mixtures of those materials.

By way of example only, a regenerated cellulose sponge may be prepared from a mixture of a viscose solution with reinforcing fibres and a porogenic compound, e.g. crystals of sodium sulphate decahydrate or of another alkali metal salt with a high content of crystallized water, the final pore dimension being related to that of the salt crystals. The viscose solution may be extruded through an extrusion die of the desired section, then let coagulate. The material is washed with water after regeneration in order to eliminate the salt and other possible soluble compounds, then it is dried and, if necessary, compressed to the desired density.

The compressed regenerated cellulose sponge has a network structure that contains air bubbles created by the elimination of the sodium sulphate crystals.

The compressed regenerated cellulose sponge material is available in various forms, e.g. in layers or sheets of different densities, thicknesses and basis weights; dry densities values for the compressed material used in the present invention are from 0.1 g/cc to 1 g/cc, while thicknesses can range from 0.2 mm to 5 mm.

The swelling upon liquid absorption of the compressed regenerated cellulose sponge material that forms the expanding layer 46 creates a void volume that does not collapse in wet conditions and therefore enables the material to rapidly acquire further releases of fluid and to transmit them to the underlying absorbent element 44 of the absorbent core 28.

In the embodiment illustrated in FIGS. 1 to 3 the total absorbent capacity of the sanitary napkin 20 is provided for by an absorbent core 28 that comprises and expanding layer 46 made of a sheet of compressed regenerated cellulose sponge having a predetermined profile in its dry state before use, and a substantially non expanding absorbent element 44.

In a preferred embodiment of the present invention, which is illustrated in FIGS. 1 and 2, the absorbent core 28 comprises an expanding layer 46 constituted by a sheet of compressed regenerated cellulose sponge with a dry density of 0.5 g/cc; the expanding layer 46 comprises in its dry state prior to use a predetermined profile along a direction that is parallel to the transverse centreline A-A, while it has no such profile in a direction parallel to the longitudinal centreline O-O. Accordingly, the expanding layer 46 has a maximum thickness of 3 mm along its longitudinal centreline O-O, which slopes towards both longitudinal side margins where a thickness of 1 mm is reached. The expanding layer 46 is positioned above the absorbent core 28 in face to face relationship with it. The absorbent layer 30 is 207 mm long and 64 mm wide, and the sheet 46 of compressed regenerated cellulose sponge is 125 mm long and 30 mm wide, being centered about both longitudinal and transverse centrelines O-O and A-A of the sanitary napkin 20. Suitable sheets of compressed regenerated cellulose sponge may be those produced by Spontex France.

The compressed regenerated cellulose sponge sheet that preferably constitutes the expanding layer 46 is capable of absorbing body fluids quickly with a large increase in its volume, generally from about 2 to 20 times, and usually from 5 to 15 times its volume at the time of the compression. The volume increase substantially corresponds to a swelling in the direction of the compression, that is in the Z-direction in the sanitary napkin 20, into the predetermined wet profile of the expanding layer 46.

The sanitary napkin 20 is produced and packaged as a conventional, substantially flat product, as illustrated in FIGS. 1 and 2. After the sanitary napkin 20 has been worn, as soon as the absorbed body fluids come in contact with the expanding layer 46, this will begin to swell in Z-direction increasing its thickness according to the predetermined profile, as can be seen in FIG. 3; the swelling is therefore more pronounced along the longitudinal centreline O-O of the sanitary napkin 20, in order to more closely conform to the user's anatomy, namely to the groove between the labia majora. The topsheet 24 follows the swelling of the expanding layer 46 by straightening out the pleats or folds 52, therefore increasing its width without restraining the swelling.

The swelling of the compressed regenerated cellulose sponge sheet that constitutes the expanding layer 46 takes place only upon activation by the absorbed fluid, that is only during the use of the sanitary napkin 20 and in close contact with the user's anatomy; the formation of the tridimensional structure can therefore achieve a much better fit with the anatomy of the user, in combination with the preferential swelling into the predetermined wet profile in the expanding layer 46. Moreover, the swelling of the compressed regenerated cellulose sponge sheet 46 may start where it is actually reached by the fluid first, for example at different positions along the longitudinal centreline O-O; the formation of the tridimensional structure may also fit, therefore, the different possible ways in which the body fluids may be released by various users.

The expanding topsheet 24 also provides a comfortable contact with the user's anatomy, without restraining the expansion of the sanitary napkin 20 into the desired tridimensional structure upon activation by body fluids.

The sanitary napkin of the present invention is preferably substantially flat prior to use, and can be therefore manufactured and packaged more easily than a conventional elasticated or pre-formed article. Since the tridimensional structure is formed only during the use, the sanitary napkin of the present invention is also easier to wear. The predetermined profile in the expanding layer 46 does not affect these features of the sanitary napkin 20 and can indeed increase the self conforming capability of the sanitary napkin 20 during the use in the period of time before the activation by body fluids and the subsequent swelling of the expanding layer 46.

The expanding layer 46 for expanding the sanitary napkin 20 into a tridimensional structure during wear may be comprised in the sanitary napkin 20 in any other suitable position and/or orientation in order to get the desired tridimensional structure, in particular, the expanding layer 46 can form at least part of the garment facing surface of the absorbent core 28.

Whenever desired, any component of the absorbent article 20 can comprise a single layer or, alternatively, multiple layers joined or affixed together to form the component itself.

In an alternate embodiment of the present invention, the sanitary napkin 20 may have two flaps (not shown), each of which are adjacent to and extend laterally from the side edge of the absorbent core. The flaps are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

The flaps may be constructed of various materials including materials used for the topsheet 24, backsheet 26, combinations thereof, and may be a laminate having tissue in the centre. Further, the flaps may be a separate element attached to the main body of the sanitary napkin 20 or can comprise extensions of the topsheet 24 and/or backsheet 26. It is recommended, however, that the flaps have a liquid impervious backsheet to prevent body fluids which reach the flaps from soiling the edges of the wearer's panties.

Preferred flaps that are suitable or adaptable to the sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the sanitary napkin 20 may comprise components that naturally wrap the sides of a wearer's panties. Sanitary napkins having components that naturally wrap the sides of a wearer's panties suitable for use with the sanitary napkin 20 of the present invention are disclosed in U.S. Patent Application Serial No. 08/096,121 entitled "Absorbent Article having Panty Covering Components that Naturally Wrap the Sides of Panties", filed July 22, 1993, in the names of Lavash, et al and U.S. Patent Application Serial No. 08/277733 entitled "Absorbent Articles Having Undergarment Covering Components with Zones of Extensibility", filed July 20, 1994, in the names of Weinberger, et al.

An optional component that can be included in the sanitary napkin 20 of the present invention is an odour control means; any suitable odour control means can be incorporated in the sanitary napkin of the present invention in any desired form, according to the techniques well known in the art.

In an alternate embodiment illustrated in FIG. 5 a sanitary napkin 20 similar to that illustrated in FIGS. 1 and 2, with an expanding layer 46 featuring the same predetermined profile as that illustrated in FIG. 2, further comprises an acquisition layer or secondary topsheet 29 positioned between the topsheet 24 and the absorbent core 28. Preferably the acquisition layer 29 does not completely overlie the absorbent core 28; in the embodiment illustrated in FIG. 5 the acquisition layer 29 does not cover the expanding layer 46 that is therefore capable of receiving the body fluids directly through the topsheet 24. As illustrated in FIG. 5 the acquisition layer 29 has a discontinuous surface comprising a window which is slightly longer and wider than the expanding layer 46; therefore the acquisition layer 29 is actually comprised between the topsheet 24 and the substantially non expanding absorbent element 44 of the absorbent core 28. Alternate configurations may also be possible, e.g. the acquisition layer 29 may comprise two narrow strips longitudinally oriented and positioned over the absorbent element 44 of the absorbent core 28 at both sides of the expanding layer 46. Alternatively, the acquisition layer 29 can be comprised between the absorbent core 28 and the backsheet 26; further, the acquisition layer 29 can be comprised between the expanding layer 46 and the absorbent element 44 in an embodiment similar to that illustrated in FIG. 2.

The acquisition layer 29 may serve several functions including improving wicking of body fluids that may escape laterally from the expanding layer 46, or, alternatively, that may reach the acquisition layer 29 directly, over and into the absorbent element 44 of the absorbent core 28. By improving wicking of body fluids, the acquisition layer 29 provides a more even distribution of the body fluids throughout the absorbent core 28.

The acquisition layer 29 preferably comprises materials that are capable of acquiring liquid very fast, and subsequently release it to contiguous layers with substantially no retention capacity.

The acquisition layer 29 may be comprised of several different materials including nonwoven or woven webs of synthetic fibres including polyester, polypropylene, or polyethylene; natural fibres including cotton or cellulose; blends of such fibres; or any equivalent materials or combinations of materials. Examples of sanitary napkins having an acquisition layer and a topsheet are more fully described in U.S. Pat. No. 4,950,264 issued to Osborn and U.S. Pat. Application Serial No. 07/810,774, "Absorbent Article Having Fused Layers", filed December 17, 1991 in the names of Cree, et al.

The topsheet 24, the acquisition layer 29 and the absorbent core 28 may also be associated in any suitable manner, in order to insure proper fluid transfer between them. In a further alternative embodiment that is not illustrated the acquisition layer 29 may be interposed between the topsheet 24 and the underlying absorbent core 28 comprising the expanding layer 46; the acquisition layer 29 must be left free to follow the expansion of the expanding layer 46 upon absorption of liquid, without restraining its swelling.

As illustrated in FIG. 5, the pleats or folds 52 are positioned at both sides of the longitudinal centreline O-O and substantially parallel to it, but in each peat or fold 52 the topsheet 24 is folded twice on itself toward the longitudinal centreline O-O of the sanitary napkin 20. During the swelling of the expanding layer 46 into the predetermined wet profile upon fluid absorption the straightening out of the pleats or folds 52 forms a sort of longitudinally oriented side cuffs 47 that provide a better seal against side leakage, as illustrated in FIG. 6; the side cuffs 47 may still be present when the swelling of the expanding layer 46 is completed if the overall width of the topsheet 24 is slightly higher than that which would be necessary to follow the complete swelling of the expanding layer 46.

Although the disposable absorbent article of the present invention has been described with reference to a sanitary napkin, it can be used beneficially in the context of other disposable absorbent articles such as panty liners, incontinence articles and diapers. The disposable absorbent article may thus also have all those features and parts which are typical for products in the context of their intended use.

## Claims

1. A disposable absorbent article for wearing adjacent a body discharge area, said article having a longitudinal centreline and a lateral centreline orthogonal thereto and defining longitudinal and lateral directions respectively, said article further having a Z-direction which is orthogonal to both said longitudinal and lateral directions, said article comprising a liquid pervious topsheet, a backsheet joined to said topsheet, an absorbent core intermediate said topsheet and said backsheet, said absorbent core comprising an expanding layer for expanding said article into a tridimensional structure while being worn by a user, said expanding layer having a body facing surface and a garment facing surface and being activated by body fluids, said absorbent article being characterized in that said expanding layer has a predetermined profile in said Z-direction in the dry state prior to use, said expanding layer being further capable, upon activation by body fluids, of expanding into a predetermined wet profile.

2. A disposable absorbent article according to claim 1, characterized in that said absorbent article is substantially flat prior to use.

3. A disposable absorbent article according to any preceding claim, characterized in that said expanding layer upon activation by body fluids expands, according to said predetermined profile, substantially in said Z-direction to provide said tridimensional structure.

4. A disposable absorbent article according to any preceding claim, characterized in that said predetermined profile is provided onto said body facing surface of said expanding layer.

5. A disposable absorbent article according to any preceding claim, characterized in that said predetermined profile of said expanding layer is provided by a non uniform thickness of said expanding layer.

6. A disposable absorbent article according to any preceding claim, characterized in that said predetermined wet profile is such that said expanding layer provides said article with a substantially convex upward configuration.

7. A disposable absorbent article according to any preceding claim, characterized in that said topsheet is capable of expanding as said absorbent article expands into a tridimensional structure upon activation by body fluids.

8. A disposable absorbent article according to any preceding claim, characterized in that said expanding layer comprises a sheet of compressed regenerated cellulose sponge having a dry density of 0.1÷1 g/cc.

9. A disposable absorbent article according to claim 8, characterized in that said sheet of compressed regenerated cellulose sponge has thicknesses between 0.2÷5 mm.

10. A disposable absorbent article according to any preceding claim, characterized in that said disposable absorbent article is a sanitary napkin or a pantiliner.
